# EUROPEAN PATENT APPLICATION

(11) **EP 2 922 048 A1**
(43) Date of publication of application: **23.09.2015**
(21) Application number: 13855297.1
(22) Date of filing: 21.05.2013
(51) Int. Cl.: G09B 9/00, G09B 23/28, A61B 1/313

(54) **HYBRID LAPAROSCOPIC SIMULATOR**

(30) Priority: 13.11.2012 RU 2012148301
(71) Applicant: Eidos-Medicine LLC, Republic of Tatarstan 420107 (RU)
(72) Inventor: VALEEV, Lenar Nailevich, Tatarstan Resp. g. Kazan 420032 (RU); ZAYNULLIN, Ramil' Khatyamovich, Tatarstan Resp. g. Kazan 420136 (RU); ANDRYASHIN, Vladimir Aleksandrovich, Tatarstan Resp. g. Kazan 420043 (RU); LITVINOV, Aleksandr Alekseevich, Tatarstan Resp. g. Kazan 420107 (RU); GAYNUTDINOV, Ramil' Talgatovich, Moscow 109341 (RU); ANDRYASHIN, Ivan Aleksandrovich, Tatarstan Resp. g. Kazan 420043 (RU); LITVINOV, Nikolay Alekseevich, Tatarstan Resp. g. Naberezhnye Chelny 423821 (RU); VALEEV, Adel' Ravil'evich, Tatarstan Resp. g. Kazan 420138 (RU); KLYUCHAROV, Igor Valer'evich, Tatarstan Resp. g. Kazan 420140 (RU); TIMOFEEV, Mikhail Evgen'evich, Moscow 117513 (RU); TSVETOV, Igor Vladimirovich, Tatarstan Resp. g. Kazan 420139 (RU); LUSHANIN, Aleksandr Viktorovich, Tatarstan Resp. g. Kazan 420140 (RU); KHAYITOV, Daniyar Dzhuraboevich, Tatarstan Resp. g. Kazan 420138 (RU)
(74) Representative: Horn Kleimann Waitzhofer Patentanwälte PartG mbB
(86) International application number: PCT/RU2013/000419
(87) International publication number: WO 2014/077732

(57) **Abstract**

The present invention provides a hybrid medical laparoscopic simulator including an ECM, laparoscopic instrument imitators coupled to the ECM, trocar imitators coupled to the ECM, a visualization system coupled to the ECM attained by the presence of a robot-patient, laparoscopic unit imitators coupled to the ECM, and operating room equipment. Each of the laparoscopic instrument imitators is executed in a form of a real instrument, contains a sensor unit, and is separated from at least four trocar imitators installed in an abdominal cavity of the robot-patient, each of the trocar imitators is coupled to a corresponding displacement node of the trocar imitator, and a position of the trocar imitator on an anterior wall of the abdominal cavity of the robot-patient is traced and defined.

## Description

### Technical Field

The invention relates to the medical educational means and is used, in particular, in the sphere of education and training of joint work of a surgical team, and of endosurgical interventions.

### Background Art

The LapSim medical simulator (manufacturer - Surgical Science, Geteborg, Sweden) contains an ECM, three trocar imitators coupled to the ECM, three laparoscopic instrument imitators coupled to the trocar imitators, a visualization system coupled to the ECM, and pedals of a coagulator coupled to the ECM. The closest analogue solution to the proposed hybrid medical laparoscopic simulator is *Computer simulator for development of manual skills of endosurgery and exercising of laparoscopic intervention methods (*LAP MENTOR, LAP MENTOR Haptic, LAP MENTOR Express models, POCC IL. MJI 13.B08453 certificate of conformity dated 21.07.2011, Simbionix, USA), containing an ECM, three trocar imitators coupled to the ECM, three laparoscopic instrument imitators coupled to the trocar imitators, a simulator's body frame containing the ECM and coupled to the trocar imitators, a visualization system coupled to the ECM, and pedals of a coagulator coupled to the ECM.

The above-described simulator does not provide the real actions and real position of a surgical team regarding a robot-patient and operational site; opportunities of different options of laparoscopic access execution in case of complicated clinical situations; complicated access (angle-wise, at a distance) according to the chosen position of the robot-patient (American, French, etc.); training of the team work of operating and assisting surgeons and scrub nurses by the different scenarios; complex training of the surgical team starting from the decision-making on the intervention on the base of the clinical record and current complaints of a virtual patient to the exercising of actions in emergency situations in the course of the intervention; and usage of more than three trocar imitators, change of the quantity of the trocar imitators, choose of the location of the trocar imitators, reality of the different instrument imitators, their insertion in the trocar imitators, and their change in the course of the intervention.

### Disclosure of the Invention

### Problem to be Solved by the Invention

Engineering task that is being solved is in the provision of the real actions and real position of a surgical team regarding a robot-patient and operational site; opportunities of different options of laparoscopic access execution in case of complicated clinical situations; complicated access (angle-wise, at a distance) according to the chosen position of the robot-patient (American, French, etc.); training of the team work of operating and assisting surgeons and scrub nurses by the different scenarios; complex training of the surgical team starting from the decision-making on the intervention on the base of the clinical record and current complaints of a virtual patient to the exercising of actions in emergency situations in the course of the intervention; and usage of more than three trocar imitators, change of the quantity of the trocar imitators, choose of the location of the trocar imitators, reality of the different instrument imitators, their insertion in the trocar imitators, and their change in the course of the intervention. Means for Solving Problem

Engineering task is being solved in the provision of a hybrid medical laparoscopic simulator including an ECM, laparoscopic instrument imitators coupled to the ECM, trocar imitators coupled to the ECM, a visualization system coupled to the ECM attained by the presence of a robot-patient, laparoscopic unit imitators coupled to the ECM, and operating room equipment, wherein each of the laparoscopic instrument imitators is executed in a form of a real instrument, contains a sensor unit, and is separated from at least four trocar imitators installed in an abdominal cavity of the robot-patient, each of the trocar imitators is coupled to a corresponding displacement node of the trocar imitator, and a position of the trocar imitator on an anterior wall of the abdominal cavity of the robot-patient is traced and defined.

The laparoscopic unit imitators are executed in the form of a coagulator control unit, pedals of a coagulator, a pedal of an aspirator-irrigator, an endovideocamera control unit, an aspirator-irrigator control unit, and an insufflator control unit with an insufflator's pipe. The laparoscopic instrument imitators are executed in the form of a laparoscopic grasper imitator, an endoscope imitator, an aspirator-irrigator imitator, a coagulator imitator, a laparoscopic scissors imitator, a dissector imitator, a hook-imitator, and a laparoscopic clip-applier imitator. The operating room equipment is executed in the form of an operating table, a surgical stand, and a side table.

### Brief Description of Drawings

[FIG. 1] FIG. 1 represents the scheme of a hybrid medical laparoscopic simulator.
[FIG. 2] FIG. 2 represents the drawing of the laparoscopic grasper that is the imitator of a laparoscopic instrument.
[FIG. 3] FIG. 3 represents the drawing of the sensor block of the laparoscopic instrument imitator.
[FIG. 4] FIG. 4 represents the drawing of the trocar imitator in section (side elevation).
[FIG. 5] FIG. 5 represents the drawing of the displacement node of the trocar imitator coupled to the trocar imitator (plan view).
[FIG. 6] FIG. 6 represents the drawing of the displacement node of the trocar imitator coupled to the trocar imitator (front elevation).
[FIG. 7] FIG. 7 schematically represents a robot-patient with trocar imitators, located in the abdominal cavity.
[FIG. 8] FIG. 8 represents a principal scheme of coupling the sensor unit microcontroller of the laparoscopic instrument imitators with the sensors of imitator of the laparoscopic instrument.
[FIG. 9] FIG. 9 represents a principal scheme of interface unit coupled to the ECM, sensor units and control units.
[FIG. 10] FIG. 10 represents a principal scheme of coupling the microcontrollers of displacement nodes of the trocar imitators to the ECM.
[FIG. 11] FIG. 11 represents a principal scheme of the coagulator control unit coupled to the sensor unit and interface unit.
[FIG. 12] FIG. 12 (12/1 and 12/2) represents a block diagram of the general algorithm of work of the ECM processor.
[FIG. 13] FIG. 13 represents a block diagram of the work algorithm of the sensor unit microcontroller of the laparoscopic instrument imitator.
[FIG. 14] FIG. 14 represents a block diagram of the work algorithm of the interface unit microcontroller.
[FIG. 15] FIG. 15 represents a block diagram of the work algorithm of the microcontrollers of the displacement nodes of the trocar imitators.
[FIG. 16] FIG. 16 represents a block diagram of the work algorithm of the main microcontroller of the displacement node of the trocar imitator.
[FIG. 17] FIG. 17 represents a block diagram of the work algorithm of the microcontroller of the coagulator control unit which is a laparoscopic unit imitator.
[FIG. 18] FIG. 18 represents a drawing of the endoscope imitator which is a laparoscopic instrument imitator.
[FIG. 19] FIG. 19 represents a principal scheme of the endovideocamera control unit coupled to the sensors' unit and interface unit.
[FIG. 20] FIG. 20 represents a principal scheme of the aspirator-irrigator control unit coupled to the interface unit.
[FIG. 21] FIG. 21 represents a principal scheme of the insufflator control unit coupled to the interface unit.
[FIG. 22] FIG. 22 represents a block diagram of the work algorithm of the microcontroller of the endovideocamera control unit.
[FIG. 23] FIG. 23 represents a block diagram of the work algorithm of the microcontroller of the aspirator-irrigator control unit.
[FIG. 24] FIG. 24 represents a block diagram of the work algorithm of the microcontroller of the insufflator control unit.

### Mode for Carrying Out the Invention

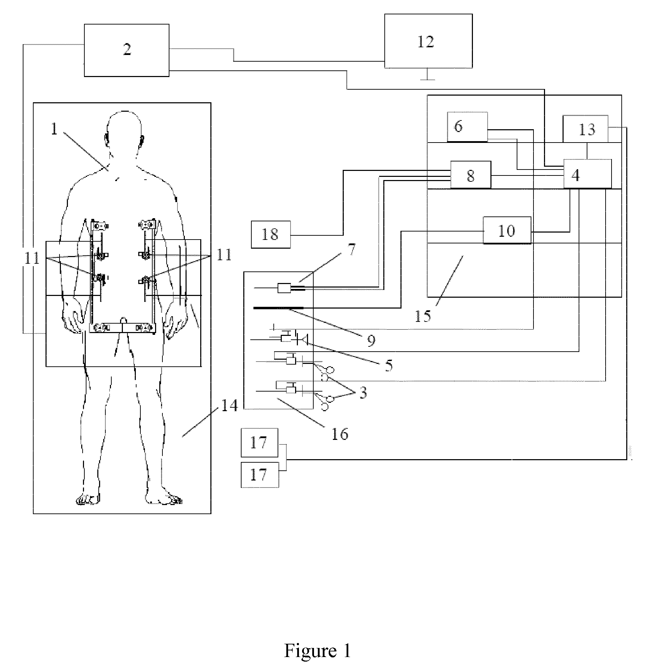

A hybrid medical laparoscopic simulator represented on FIG. 1 includes: robot-patient 1, ECM (electronic computing machine) 2, laparoscopic grasper imitators 3, which are the laparoscopic instruments imitators, coupled to the ECM 2 through interface unit 4, endoscope imitator 5, which is the laparoscopic instrument imitator, coupled to the ECM 2 through endovideocamera control unit 6 and interface unit 4, aspirator-irrigator imitator 7, which is the laparoscopic instrument imitator, coupled to the ECM 2 through aspirator-irrigator control unit 8 and interface unit 4, insufflator pipe 9 coupled to insufflator control unit 10, trocar imitators 11, with their quantity of more than three (four in this execution), installed in the abdominal cavity of the robot-patient 1 and coupled to the ECM 2, visualization system 12 coupled to the ECM 2, interface unit 4 coupling the ECM 2 to the laparoscopic graspers imitators 3, coagulator control unit 13, which is one of the laparoscopic unit imitators, endovideocamera control unit 6, which is one of the laparoscopic unit imitators, aspirator-irrigator control unit 8, which is one of the laparoscopic unit imitators, and insufflator control unit 10, which is one of the laparoscopic unit imitators. FIG. 1 represents the operating room equipment in the form of: operating table 14, surgical stand 15, and side table 16. FIG. 1 represents laparoscopic instrument imitators in the form of: laparoscopic grasper imitators 3, endoscope imitator 5, and aspirator-irrigator imitator 7. FIG. 1 represents the laparoscopic unit imitators in the form of endovideocamera control unit 6, aspirator-irrigator control unit 8, insufflator control unit 10 with insufflator pipe 9, coagulator control unit 13, pedals of coagulator 17, and a pedal of aspirator-irrigator 18.

Trocar imitators 11 are located in the abdominal cavity of the robot-patient 1, robot-patient 1 is located on the operating table 14, visualization system 12 is coupled to the ECM 2, interface unit 4 is coupled to the ECM 2, laparoscopic graspers imitators 3 are coupled to the interface unit 4, endoscope imitator 5 is coupled to the endovideocamera control unit 6, aspirator-irrigator imitator 7 is coupled to the aspirator-irrigator control unit 8, and insufflator pipe 9 is coupled to the insufflator control unit 10. Laparoscopic grasper imitators 3, endoscope imitator 5, aspirator-irrigator imitator 7, and insufflator pipe 9 are located on the side table 16. Coagulator control unit 13, endovideocamera control unit 6, aspirator-irrigator control unit 8, and insufflator control unit 10 are coupled to the interface unit 4. Pedals of coagulator 17 are coupled to the coagulator control unit 13 and a pedal of aspirator-irrigator 18 is coupled to the aspirator-irrigator control unit 8.

The circuit design of the ECM 2 could be implemented as *IntelCorei7* processor with the frequency of 3500 MHz, *Kingston* RAM such as DDR3 with the storage space of 8 GB, *NVIDIA Ge Force GTX560* video card with the storage space of 2 GB, *Seagate* hard disk drive with the storage space of 500 GB, and *Microsoft Windows 7 Processional* operating system. ECM 2 contains a pointing device allowing input the data to the program running in the processor of the ECM 2.

Robot-patient 1 can be realized on the model of HPS robot-simulator supplied by *Virtumed LLC,* www.virtumed.ru.

Visualization system 12 can be realized on the model of TS 1716L-6(S/U) 17" LCD, *Neovo X*-*19AV White* display, supplied by *ELLIPS Partner LLC.*

Operating table 14 can be realized on the model of *StarTech 3008C* supplied by the *Delrus Kazan LLC.*

Surgical stand 15 contains 5 shelves and can be realized on the model of Spya-03-05-KMT supplied by *FinStar LLC.*

Side table 16 can be realized on the model of *Goose* table supplied by *Belaya Mebel LLC.*

Pedals of coagulator 17 can be realized on the model of two-keyed pedal for ESHF A-001 supplied by *ELLIPS Partner LLC.*

A pedal of aspirator-irrigator 18 can be realized on the model of one-keyed pedal of aspirator-irrigator supplied by *ELLIPS Partner LLC.*

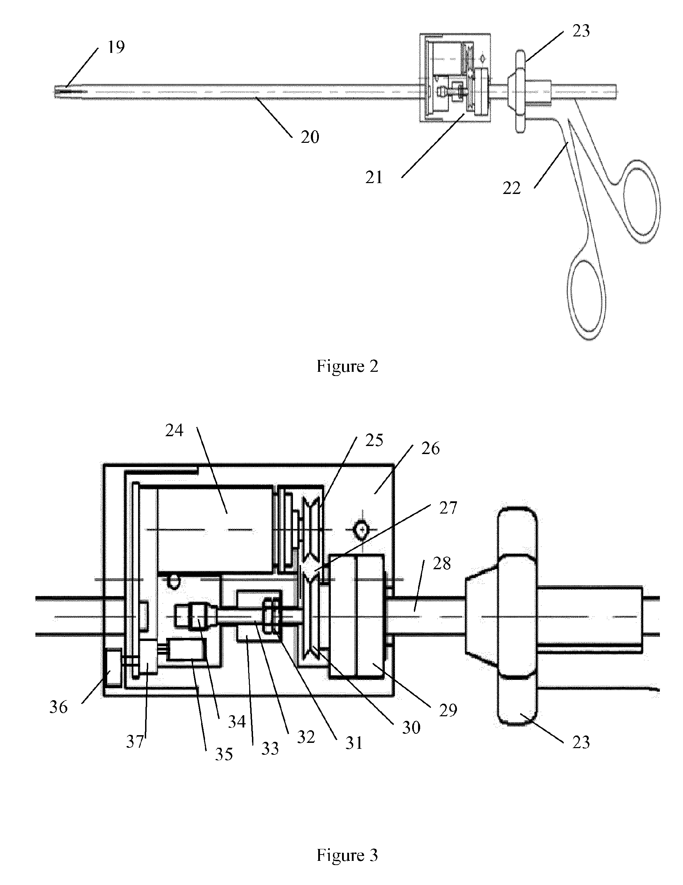

Laparoscopic grasper imitator 3, which is a laparoscopic instrument imitator, represented on FIG. 2 drawing includes: branches 19, rod 20, sensor unit 21, handle 22, and instrument wing nut 23. Branches 19 are coupled to the rod 20, rod 20 is coupled to the sensor unit 21, handle 22 is coupled to the instrument wing nut 23, and instrument wing nut 23 is coupled to the sensor unit 21.

Sensor unit 21 of the laparoscopic instrument imitator represented on FIG. 3 drawing includes: instrument wing nut 23, encoder 24, encoder pulley 25, sensor unit body frame 26, belt 27, pipe 28, bearing 29, handle pulley 30, lock ring 31, tie 32, stroke limitation cage 33, magnetic head 34, Hall sensor 35, infrared LED 36, and sensor unit microcontroller 37. Grooves for installation of encoder 24, Hall sensor 35, infrared LED 36, sensor unit microcontroller 37, handle pulley 30, encoder pulley 25, and bearing 29 are provided in the sensor unit body frame 26. Pipe 28 is coupled to the bearing 29, tie 32 is clenched with the lock ring 31, magnetic head 34 is fixed on the end of the tie 32, handle pulley 30 is set onto the pipe 28, encoder pulley 25 is set on the axis of the encoder 24, and encoder pulley 25 and handle pulley 30 are coupled with the belt 27. Hall sensor 35, encoder 24, infrared LED 36, and sensor unit microcontroller 37 are coupled to the sensor unit body frame 26. Encoder 24 can be realized on the model of LIR212A, produced by *SKB IS,* Saint-Petersburg.

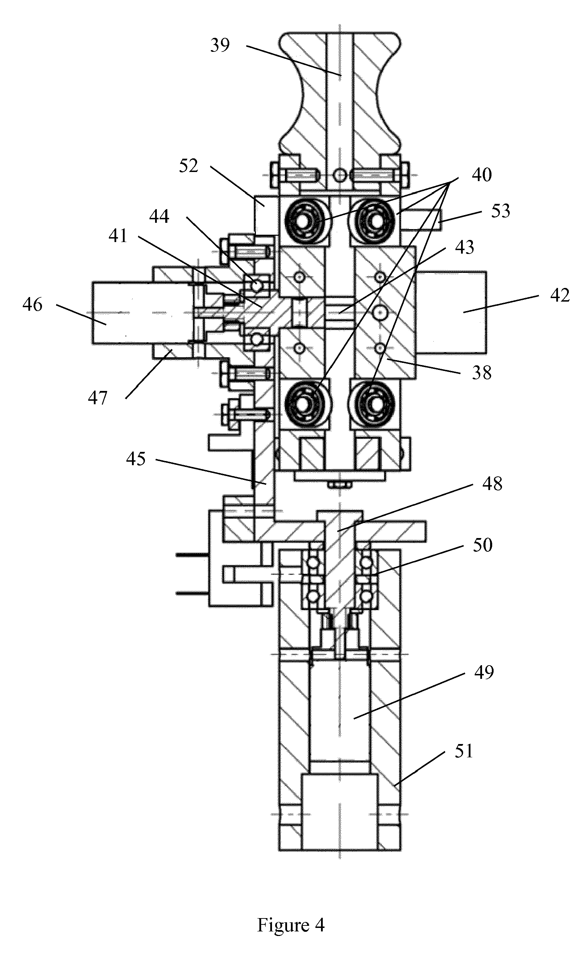

Trocar imitator 11 represented on FIG. 4 drawing in section (side elevation) includes: trocar imitator body frame 38, laparoscopic instrument receptacle 39, holding rollers 40, trocar imitator body frame shaft 41, instrument longitudinal motion detection sensor 42, which can be realized on the model of EMS22 produced by *Bourns,* Columbia, www.bourns.com, instrument longitudinal motion detection sensor shaft 43, shaft bearing 44, movable angle bar 45, Y-direction rotation sensor 46, which can be realized on the model of LIR212A, Y-direction rotation sensor catch 47, movable angle bar shaft 48, X-direction rotation sensor 49, which can be realized on the model of LIR212A, movable angle bar bearing 50, holding body frame 51, infrared detector 52, and trocar valve 53. Trocar imitator 38 is coupled to the laparoscopic instrument receptacle 39, trocar imitator body frame 38 is coupled to the holding rollers 40, trocar imitator body frame 38 is coupled to the trocar imitator body frame shaft 41, trocar imitator body frame 38 is coupled to the instrument longitudinal motion detection sensor 42, the instrument longitudinal motion detection sensor 42 is coupled to the instrument longitudinal motion detection sensor shaft 43, shaft bearing 44 is coupled to the trocar imitator body frame shaft 41, shaft bearing 44 is coupled to the movable angle bar 45, Y-direction rotation sensor 46 is coupled to the trocar imitator body frame shaft 41, Y-direction rotation sensor 46 is coupled to the Y-direction rotation sensor catch 47, Y-direction rotation sensor catch 47 is coupled to the movable angle bar 45, movable angle bar 45 is coupled to the movable angle bar shaft 48, movable angle bar shaft 48 is coupled to the X-direction rotation sensor 49, movable angle bar shaft 48 is coupled to the movable angle bar bearing 50, holding body frame 51 is coupled to the movable angle bar bearing 50, holding body frame 51 is coupled to the X-direction rotation sensor 49, infrared detector 52 is coupled to the trocar imitator body frame 38, and trocar valve 53 is coupled to the trocar imitator body frame 38.

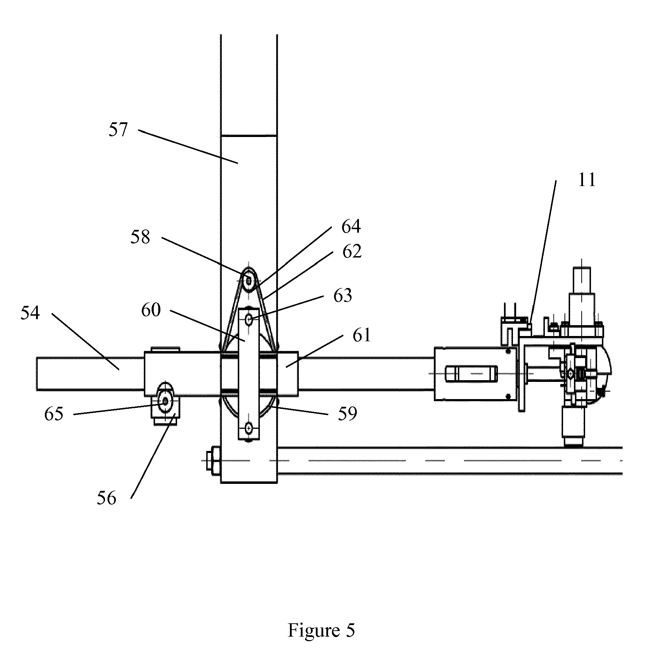

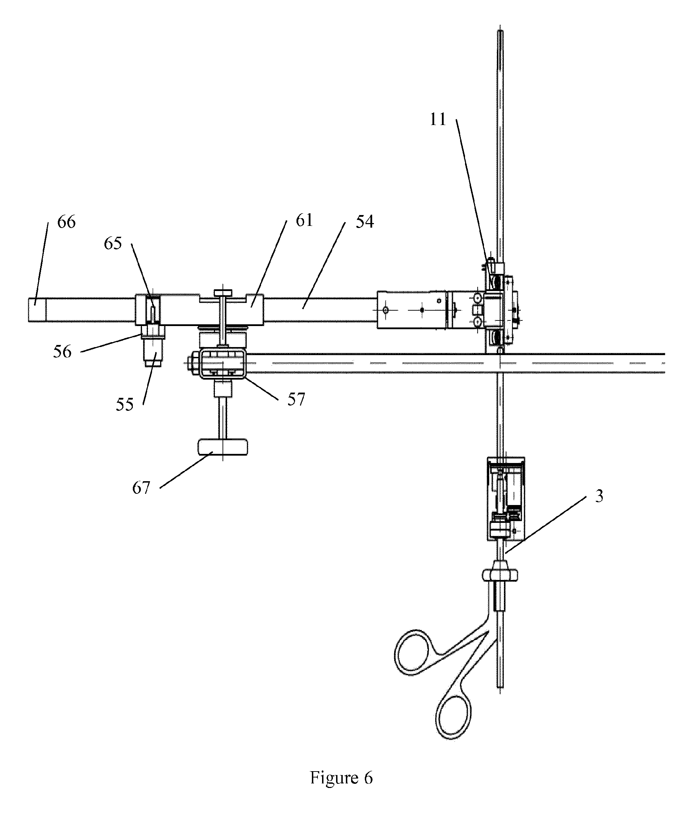

Displacement node of the trocar imitator coupled to the trocar imitator 11 (plan view and front elevation) represented on FIG. 5 and FIG. 6 drawings includes: guideway 54, guideway longitudinal motion sensor 55, longitudinal motion sensor catch 56, connecting strap 57, guideway rotation sensor 58, guideway pulley 59, guideway body frame catch 60, guideway body frame 61, rubber ring 62, guideway body frame catch pins 63, sensor pulley 64, silicone shaft 65, trocar imitator 11, laparoscopic grasper imitator 3, microcontroller of displacement node of the trocar imitators 66 (66₁-66₃ - microcontroller of displacement nodes of the trocar imitators, 66₄ - main microcontroller of displacement node of the trocar imitators), and displacement node wing nut 67. Laparoscopic grasper imitator 3 is inserted in the trocar imitator 11, trocar imitator 11 is coupled to the guideway 54, guideway 54 is inserted in the guideway body frame 61, guideline body frame catch 60 is coupled to the connecting strap 57 with the help of the guideway body frame catch pins 63, silicone shaft 65 is pressed against the guideline 54, longitudinal motion sensor catch 56 is coupled to the guideway body frame 61, guideway longitudinal motion sensor 55 is coupled to the silicone shaft 65, guideway longitudinal motion sensor 55 is coupled to the longitudinal motion sensor 56, guideway pulley 59 and sensor pulley 64 are coupled with the rubber ring 62, guideway pulley 59 is coupled to the guideway 54, and sensor pulley 64 is coupled guideway rotation sensor 58. Guideway 54 is a hollow aluminum tube of square section, inside of the tube signal wires coupling microcontroller of displacement node of the trocar imitator 66 to the instrument longitudinal motion detection sensor 42, the Y-direction rotation sensor 46, the X-direction rotation sensor 49, and the infrared detector 52 are laid.

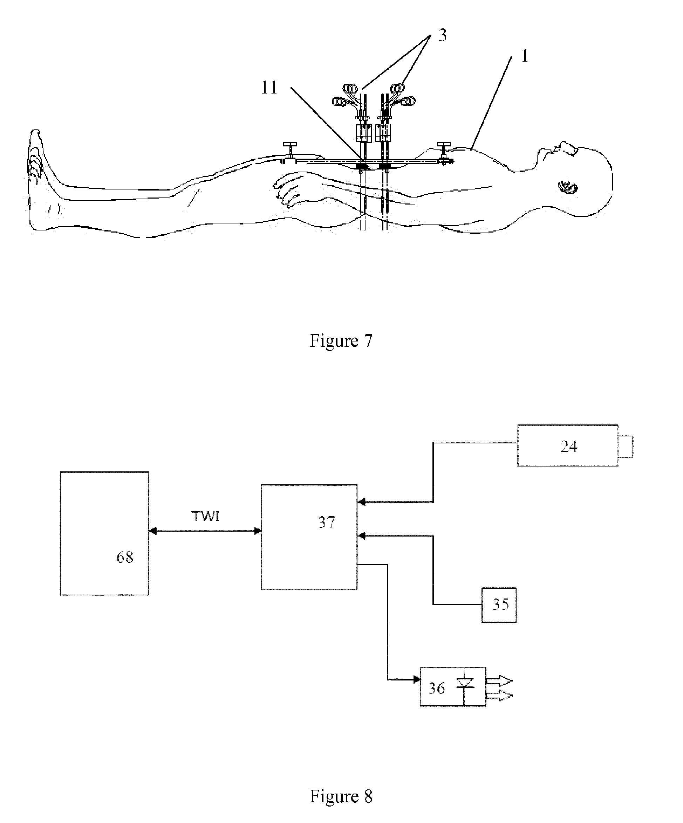

Robot-patient 1 with trocar imitators 11 located in its abdominal cavity schematically represented on FIG. 7 drawing includes: robot-patient 1, laparoscopic grasper imitators 3, and trocar imitators 11. Trocar imitators 11 are located in the abdominal cavity of the robot-patient 1 and laparoscopic grasper imitators 3 are inserted in the trocar imitators 11.

Principal scheme of connection of the sensor unit microcontroller of the imitator of the laparoscopic instruments with the sensors of imitator of the laparoscopic instrument represented on FIG. 8 drawing includes: sensor unit microcontroller 37, which can be realized on the model of AtMega8and is located in the sensor unit 21 represented on FIG. 3, encoder 24, which can be executed on the model of LIR212, Hall sensor 35, which can be executed on the model of SS49, infrared LED 36, and interface unit microcontroller 68. Sensor unit microcontroller 37 is coupled to the Hall sensor, the encoder 24, the interface unit microcontroller 68, and the infrared LED 36 respectively.

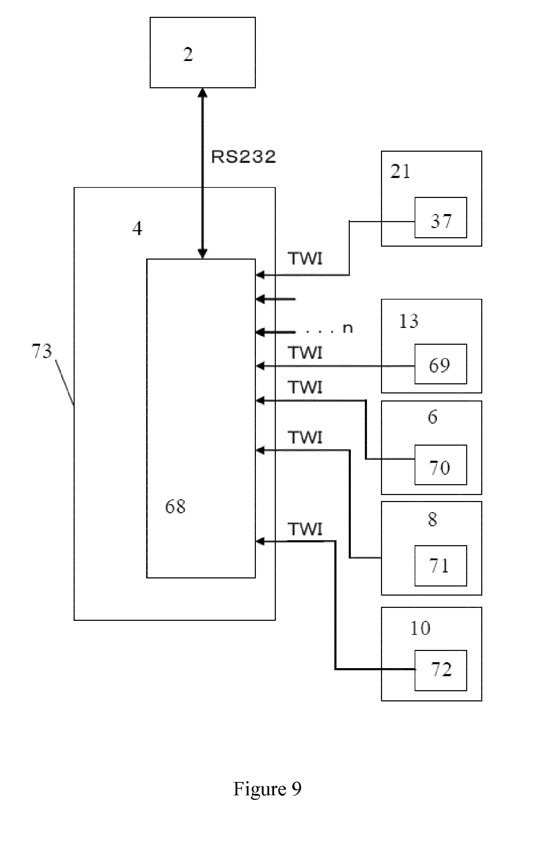

Principal scheme of interface unit 4 coupled to the sensor units 21 and control units represented on FIG. 9 drawing includes: the ECM 2, sensor unit 21, interface unit 4, coagulator control unit 13, endovideocamera control unit 6, aspirator-irrigator control unit 8, insufflator control unit 10, n sensor unit microcontrollers 37 (n is a number of the coupled laparoscopic instrument imitators), for example, n can assume a value equal to four, interface unit microcontroller 68, which can be realized on the model of AtMegal6, coagulator control unit microcontroller 69, which can be realized on the model of AtMega16, endovideocamera control unit microcontroller 70, which can be realized on the model of AtMega16, aspirator-irrigator control unit microcontroller 71, which can be realized on the model of AtMega16, insufflator control unit microcontroller 72, which can be realized on the model of AtMega16, and interface unit body frame 73. Interface unit microcontroller 68 is coupled to the n-number of the sensor unit microcontrollers 37 (one sensor unit microcontroller 37 is represented on FIG. 9), the coagulator control unit microcontroller 69, the endovideocamera control unit microcontroller 70, the aspirator-irrigator control unit microcontroller 71, the insufflator control unit microcontroller 72, and the ECM 2 respectively. Interface unit microcontroller 68 is located inside the interface unit body frame 73.

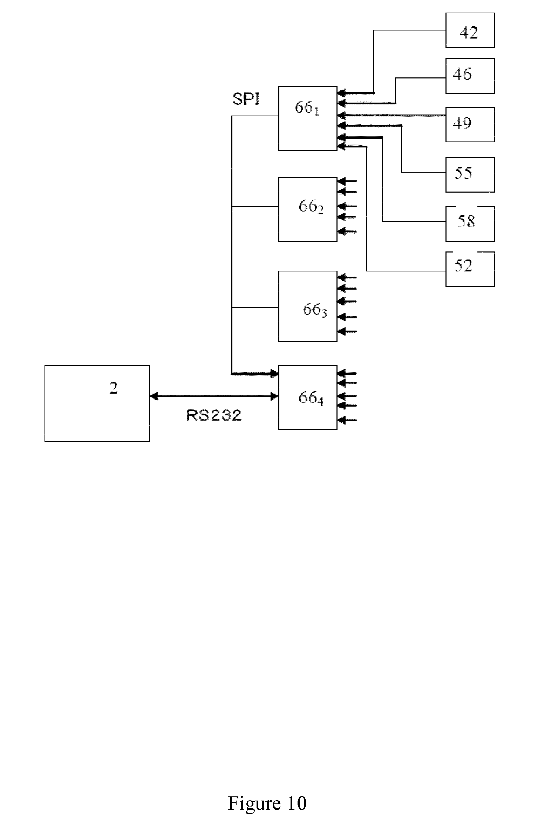

Principal scheme of coupling the microcontrollers of displacement node of the trocar imitators 66 to the ECM 2 represented on FIG. 10 drawing includes: the ECM 2, microcontrollers of displacement node of the trocar imitators 66 (66₁, 66₂, 66₃, 66₄), which can be realized on the model of STM32 F100MB, the instrument longitudinal motion detection sensor 42, Y-direction rotation sensor 46, X-direction rotation sensor 49, guideway longitudinal motion sensor 55, guideway rotation sensor 58, and infrared detector 52. The microcontroller of displacement node of the trocar imitator 66₄ is coupled to the ECM 2. The microcontroller of displacement node of the trocar imitator 66₁ is coupled to the instrument longitudinal motion detection sensor 42, the Y-direction rotation sensor 46, the X-direction rotation sensor 49, the guideway longitudinal motion sensor 55, the guideway rotation sensor 58, and the infrared detector 52. Every microcontroller of displacement node of the trocar imitator 66₂-66₄ is realized analogously to the microcontroller of displacement node of the trocar imitator 66₁ and is coupled to the outputs of the instrument longitudinal motion detection sensor 42, the Y-direction rotation sensor 46, the X-direction rotation sensor 49, the guideway longitudinal motion sensor 55, the guideway rotation sensor 58, and the infrared detector 52 respectively (are not represented on FIG. 10).

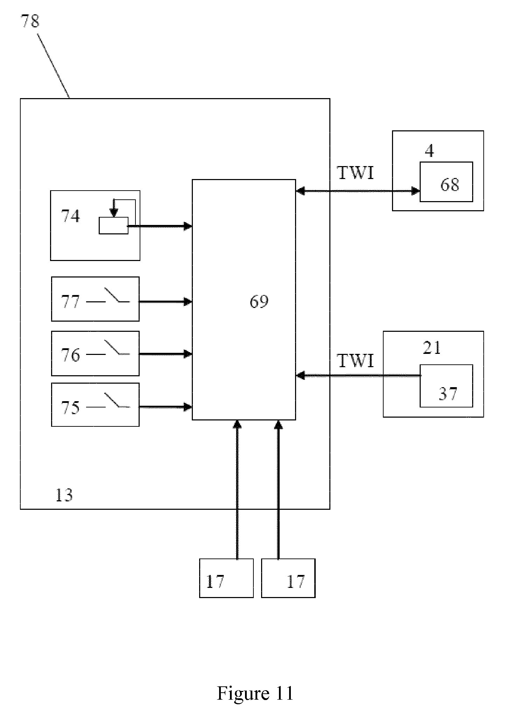

Principal scheme of the coagulator control unit 13, coupled to the sensor unit 21 and interface unit 4, represented on FIG. 11 includes: coagulator control unit 13, pedals of the coagulator 17, sensor unit microcontroller 37, interface unit microcontroller 68, coagulator control unit microcontroller 69, coagulator's current power controller 74, coagulation mode switch 75, cutting mode switch 76, blend mode switch 77, sensor unit 21, interface unit 4, and coagulator control unit body frame 78.

Coagulator control unit microcontroller 69 is coupled to the pedals of the coagulator 17, the coagulator's current power controller 74, the coagulation mode switch 75, the cutting mode switch 76, the blend mode switch 77, the interface unit microcontroller 68, and the sensor unit microcontroller 37. Coagulator's current power controller 74, coagulation mode switch 75, cutting mode switch 76, and blend mode switch 77 are located on the coagulator control unit body frame 78. Coagulator control unit microcontroller 69 is located inside the coagulator control unit body frame 78.

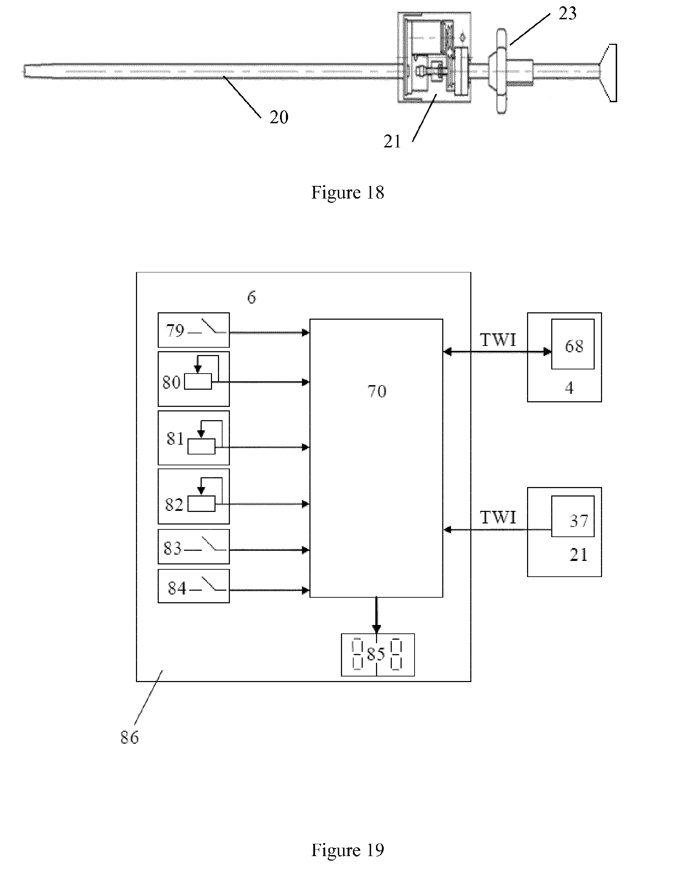

Endoscope imitator 5, which is the laparoscopic instrument imitator, represented on FIG. 18 drawing includes: rod 20, sensor unit 21,and instrument wing nut 23. Rod 20 is coupled to the sensor unit 21 and instrument wing nut 23 is coupled to the sensor unit 21.

Principal scheme of endovideocamera control unit 6, coupled to the sensor unit 21 and interface unit 4, represented on FIG. 19 includes: endovideocamera control unit 6, sensor unit 21, interface unit 4, endovideocamera control unit microcontroller 70, endovideocamera switch 79, color phase controller 80, illuminance controller 81, white-balance controller 82, halogen light switch 83, xenon light switch 84, brightness display 85, which can be realized on the model of BAS6-11EWA, sensor unit microcontroller 37, interface unit microcontroller 68, and endovideocamera control unit body frame 86. Endovideocamera switch 79, color phase controller 80, illuminance controller 81, white-balance controller 82, halogen light switch 83, xenon light switch 84, and brightness display 85 are installed on the endovideocamera control unit body frame 86. Endovideocamera control unit microcontroller 70 is coupled to the endovideocamera switch 79, the color phase controller 80, the illuminance controller 81, the white-balance controller 82, the halogen light switch 83, the xenon light switch 84, the sensor unit microcontroller 37, the interface unit microcontroller 68, and the brightness display 85. Endovideocamera control unit microcontroller 70 is located inside the endovideocamera control unit body frame 86.

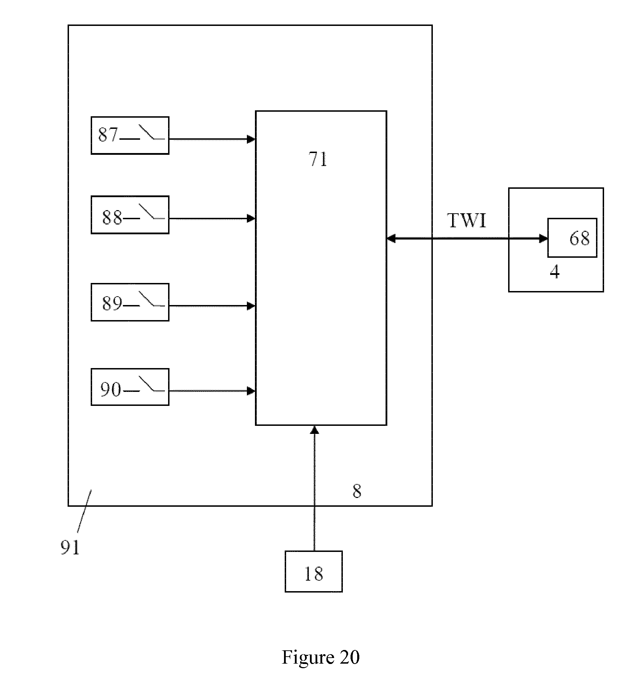

Principal scheme of the aspirator-irrigator control unit 8 coupled to the interface unit 4 represented on FIG. 20 includes: aspirator-irrigator control unit 8, interface unit 4, aspirator-irrigator control unit microcontroller 71, a pedal of the aspirator-irrigator 18, aspirator-irrigator switch 87, aspiration mode switch 88, irrigation mode switch 89, opening and closing switch 90, interface unit microcontroller 68, and aspirator-irrigator control unit body frame 91. Aspirator-irrigator control unit microcontroller 71 is coupled to the pedal of the aspirator-irrigator 18, the aspirator-irrigator switch 87, the aspiration mode switch 88, the irrigation mode switch 89, the opening and closing switch 90, and the interface unit microcontroller 68. Aspirator-irrigator switch 87, aspiration mode switch 88, irrigation mode switch 89, and opening and closing switch 90 are located on the aspirator-irrigator control unit body frame 91. The aspirator-irrigator control unit microcontroller 71 is located inside the aspirator-irrigator control unit body frame 91.

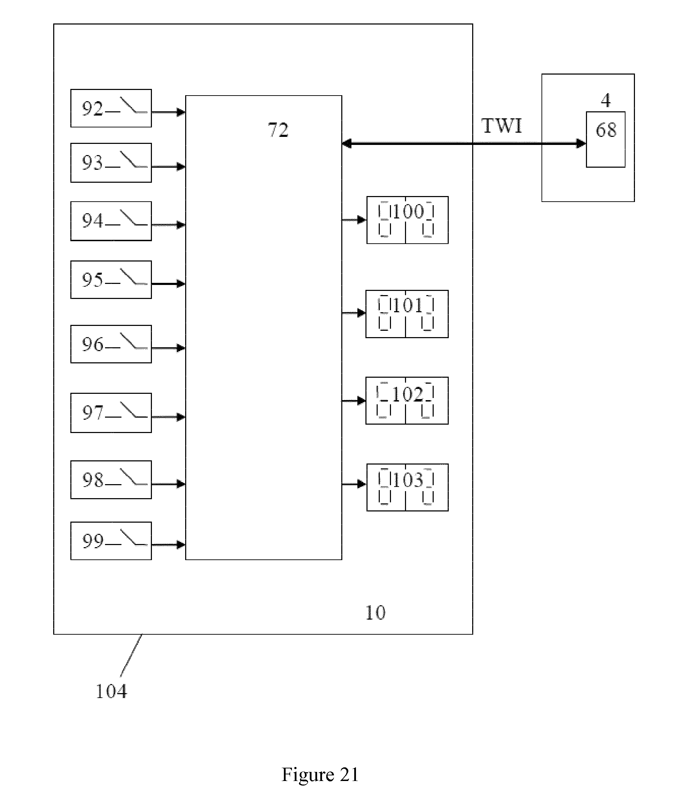

Principal scheme of the insufflator control unit 10 coupled to the interface unit 4 represented on FIG. 21 includes: insufflator control unit 10, interface unit 4, insufflator control unit microcontroller 72, interface unit microcontroller 68, insufflator switch 92, insufflation start button 93, pressure increase switch 94, pressure decrease switch 95, pressure memorization switch 96, flow increase switch 97, flow decrease switch 98, flow memorization switch 99, set pressure display 100, measured pressure display 101, set flow display 102, measured flow display 103, and insufflator control unit body frame 104. Insufflator control unit microcontroller 72 is coupled to the outputs of the interface unit microcontroller 68, the insufflator switch 92, the insufflation start button 93, the pressure increase switch 94, the pressure decrease switch 95, the pressure memorization switch 96, the flow increase switch 97, the flow decrease switch 98, and the flow memorization switch 99 and the inputs of the set pressure display 100, the measured pressure display 101, the set flow display 102, and the measured flow display 103. Insufflator switch 92, insufflation start button 93, pressure increase switch 94, pressure decrease switch 95, pressure memorization switch 96, flow increase switch 97, flow decrease switch 98, flow memorization switch 99, set pressure display 100, measured pressure display 101, set flow display 102, and measured flow display 103 are installed on the insufflator control unit body frame 104. Insufflator control unit microcontroller 72 is located inside the insufflator control unit body frame 104.

Let us consider laparoscopic grasper imitator 3, which is the laparoscopic instrument imitator, represented on FIG. 2 drawing and sensor unit 21 of the laparoscopic instrument imitator represented on FIG. 3 drawing, in operation. As a surgeon rotates the wing nut 23 on the handle 22, the rotation is communicated to the encoder 24 through the handle pulley 30 and encoder pulley 25. Thus the rotation tracking of the rod 20 of the laparoscopic grasper imitator 3 occurs. The span of the branches 19 is tracked by the use of Hall sensor 35. Hall sensor 35 determines the position of the magnetic head 34 fixed on the tie 32. When operating the handle 22, tie 32 executes longitudinal motions being registered by Hall sensor 35. Travel of the handle 22 is limited by the lock ring 31, which bears against the walls of the stroke limitation cage 33, thus the real stroke limitation of the travel of the branches 19 is imitated. Detailed description of the surgeon's manipulations with the help of the laparoscopic grasper imitator 3 and other laparoscopic instrument imitators is examined on pages from twenty-two to forty-three, where the hybrid medical laparoscopic simulator represented on FIG. 1 scheme and in the examples of *Cholecystectomy* and *Adnexectomy* intervention training is examined in operation.

Let us consider trocar imitator 11, represented on FIG. 4 drawing in section (side elevation), in operation. Laparoscopic instrument imitator, for example, laparoscopic grasper imitator 3, which is one of the laparoscopic instruments, is inserted in the vent of the laparoscopic instrument receptacle 39. Laparoscopic grasper imitator 3 is being rotated in the X-and Y-direction as in the course of the real intervention execution, laparoscopic instrument receptacle 39 and trocar imitator bode frame 38 are being rotated respectively, the rotation is communicated to the Y-direction rotation sensor 46 through the trocar imitator body frame shaft 41, and the rotation is communicated to the X-direction rotation sensor 49 as well through the movable angle bar 45 and the movable angle bar shaft 48. Y-direction rotation sensor 46 and X-direction rotation sensor 49 are tracking the coordinates of position in space of the laparoscopic grasper imitator 3. When being inserted, laparoscopic grasper imitator 3 touches the holding rollers 40 and the instrument longitudinal motion detection sensor shaft 43, and instrument longitudinal motion detection sensor 42 registers the depth of insertion of the laparoscopic grasper imitator 3 in the laparoscopic instrument receptacle 39 thereby. Detailed description of the surgeon's manipulations when installing the trocar imitators 11 into the abdominal cavity of the robot-patient 1 is examined on pages twenty-six and thirty-five, in the examples of *Cholecystectomy* and *Adnexectomy* intervention training.

Let us consider displacement node of the trocar imitator 11 coupled to the trocar imitator 11 (plan view and front elevation) represented on FIG. 5 and FIG. 6, in operation. Guideway 54 allows the surgeon to set the required for the work position of the trocar imitators 11. The surgeon slackens the displacement node wing nut 67, chooses the rotation angle and the distance of the trocar imitators 11 form the node, then tightens the displacement node wing nut 67. Rotation of the guideway 54 is communicated through the guideway pulley 59 and sensor pulley 64 to the guideway rotation sensor 58. Guideway longitudinal motion sensor 55 defines longitudinal motion of the guideway 54. Thus the position of the trocar imitators 11 on the anterior wall of the abdominal cavity of the robot-patient 1 is defined.

Let us consider robot-patient 1 with the trocar imitators located in the abdominal cavity, schematically represented on FIG. 7, in operation. Trocar imitators 11 are installed into the robot-patient 1 and are located in its abdominal cavity. There are four movable trocar imitators 11 in this execution of the hybrid medical laparoscopic simulator. According to the type of the endosurgical intervention, different positions of the trocar imitators 11 are being set. Different types of laparoscopic instrument imitators are being used that include endoscope imitator 5, a coagulator imitator, a laparoscopic scissors imitator, laparoscopic grasper imitator 3, a dissector imitator, a hook imitator, a laparoscopic clip applier imitator, aspirator-irrigator imitator 7, etc. A coagulator imitator is realized by analogy to the laparoscopic grasper imitator 3.

Let us consider the principal scheme of coupling the sensor unit microcontroller 37 of the laparoscopic instrument imitator with the sensors of imitator of the laparoscopic instrument represented on FIG. 8, in operation.

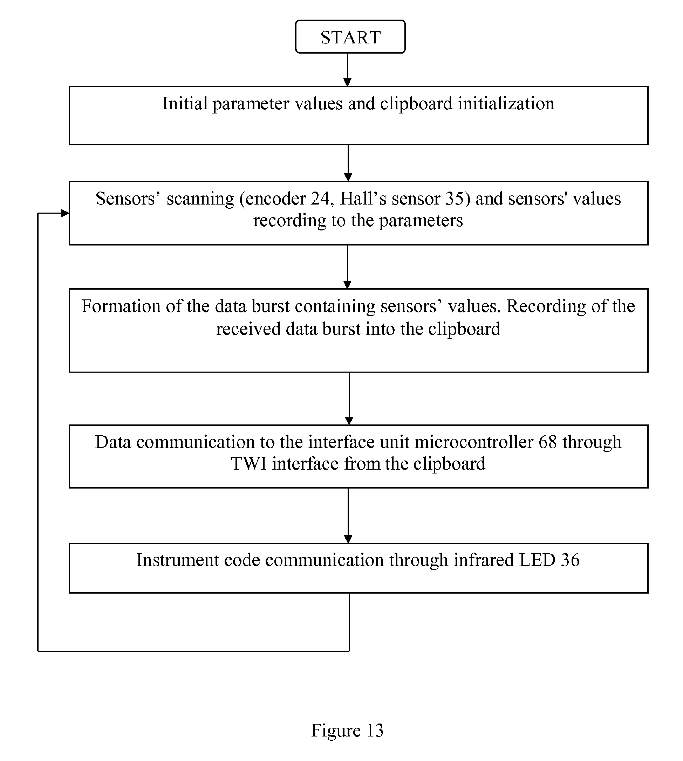

The work algorithm of the sensor unit microcontroller 37 of the laparoscopic instrument imitator is represented on FIG. 13 block diagram. Data from the encoder 24 and the Hall sensor 35, tracking the coordinates of the span angle of the branches 19 and of the rotation of the laparoscopic instrument imitator 3, represented on FIG. 2 proceeds to the sensor unit microcontroller 37 that converts the data into data bursts being sent through the TWI interface to the interface unit microcontroller 68 and being detected by the interface unit microcontroller 68. Data from the sensor unit microcontroller 37 is communicated to the infrared LED 36, that proceeds the signal with the instrument code to the infrared detector 52, located in the trocar imitator 11, represented on FIG. 4 drawing (thus the system detects the type of the laparoscopic instrument imitator inserted in the certain trocar imitator 11).

Let us consider the principal scheme of interface unit 4 coupled to the sensor units 21 and control units represented on FIG. 9, in operation. Sensor unit microcontrollers 37 of the laparoscopic instrument imitator, coagulator control unit microcontroller 69, which is located in the coagulator control unit 13, endovideocamera control unit microcontroller 70, which is located in the endovideocamera control unit 6, aspirator-irrigator control unit microcontroller 71, which is located in the aspirator-irrigator control unit 8, and insufflator control unit microcontroller 72, which is located in the insufflator control unit 10, are coupled to the interface unit microcontroller 68, which is located in the interface unit 4 through the TWI interface.

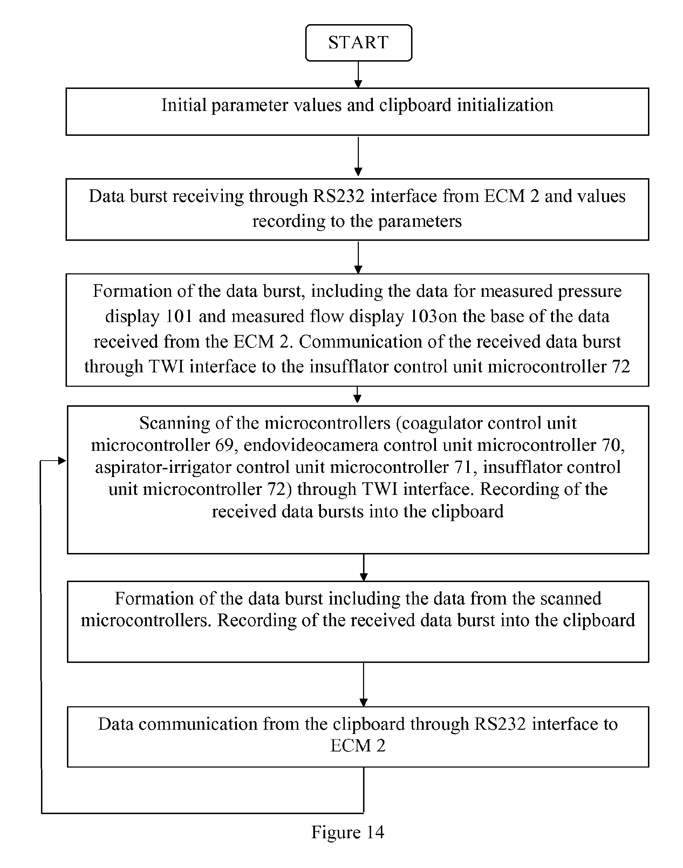

The work algorithm of the interface unit microcontroller 68 is represented on FIG. 14 block diagram. Interface unit microcontroller 68 consequently requests the data from n-number of the sensor unit microcontrollers 37 (n is the number of the coupled laparoscopic instrument imitators), the endovideocamera control unit microcontroller 70, the aspirator-irrigator control unit 71, insufflator control unit microcontroller 72, and coagulator control unit microcontroller 69 through the TWI interface. Interface unit microcontroller 68 converts the received data to data bursts detected by the program on the ECM 2. The data are being communicated to the ECM 2 through RS232 COM-port.

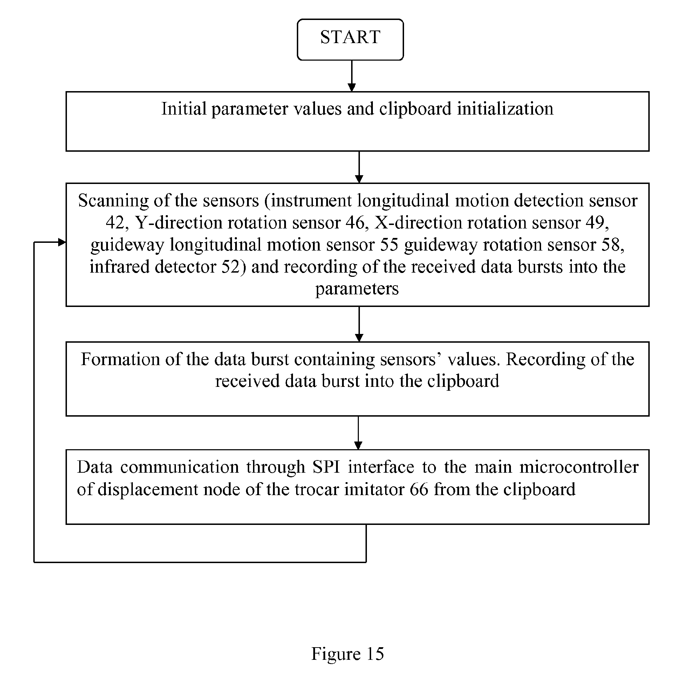

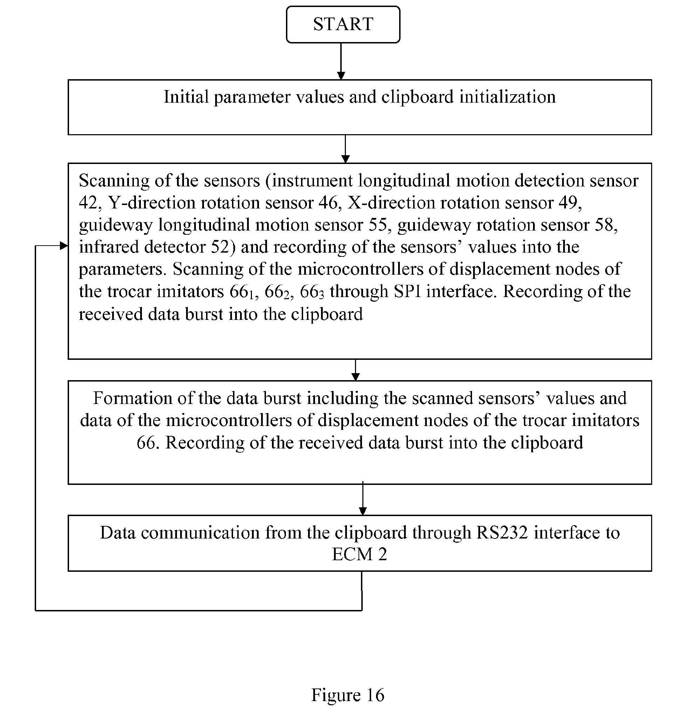

Let us consider the principal scheme of coupling the microcontrollers of displacement nodes of the trocar imitators 66 to the ECM 2 represented on FIG. 10, in operation. FIGs. 15 and 16 each represent a block diagram of the work algorithm of the microcontrollers of the displacement nodes of the trocar imitators 66. Data from the instrument longitudinal motion detection sensor 42, the Y-direction rotation sensor 46, the X-direction rotation sensor 49, the guideway rotation sensor 58, the guideway longitudinal motion sensor 55 tracking the position change of the trocar imitators 11 in space, and the infrared detector 52 that receives the signal from the infrared LED 36 and detects the type of the laparoscopic instrument imitator inserted in the trocar imitator 11 proceed to the microcontrollers of displacement nodes of the trocar imitators 66 (66₁, 66₂, 66₃, 66₄) with a similar set of the above-mentioned sensors for each microcontroller of displacement nodes of the trocar imitators 66. This execution of the hybrid medical laparoscopic simulator contains 4 movable trocar imitators 11 and 4 microcontrollers of displacement nodes of the trocar imitators 66 represented on FIG. 10 correspondingly. The main microcontroller of displacement node of the trocar imitators 66₄ (master) consequently requests the data via SPI interface from the other three displacement node of the trocar imitators 66₁-66₃, converts all the data received into one data burst recognized by the program of the ECM 2, and through the RS232 serial port communicates the data to the ECM 2.

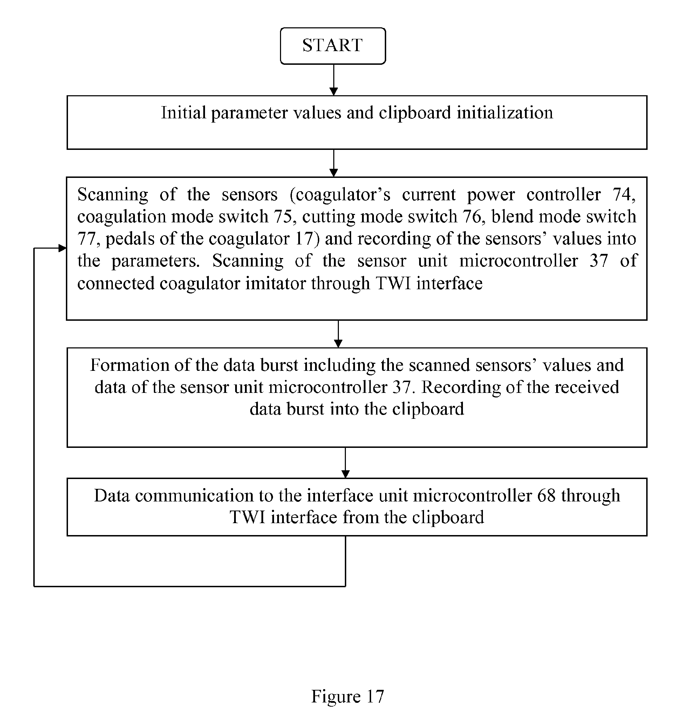

Let us consider the principal scheme of the coagulator control unit 13 coupled to the sensor unit 21 and interface unit 4 represented on FIG. 11, in operation. FIG. 17 represents a block diagram of the work algorithm of the microcontroller of the coagulator control unit 69 which is a laparoscopic unit imitator. The microcontroller of the coagulator control unit 69 is located inside the coagulator control unit 13. Data from the coagulator's current power controller 74, the coagulation mode switch 75, the cutting mode switch 76, and the blend mode switch 77 are communicated to the microcontroller of the coagulator control unit 69. Data from the sensor unit microcontroller 37 located in the coagulator imitator that is the laparoscopic instrument imitator are communicated to the microcontroller of the coagulator control unit 69 through TWI interface. The microcontroller of the coagulator control unit 69 is communicating the collected data through TWI interface to the interface unit microcontroller 68.

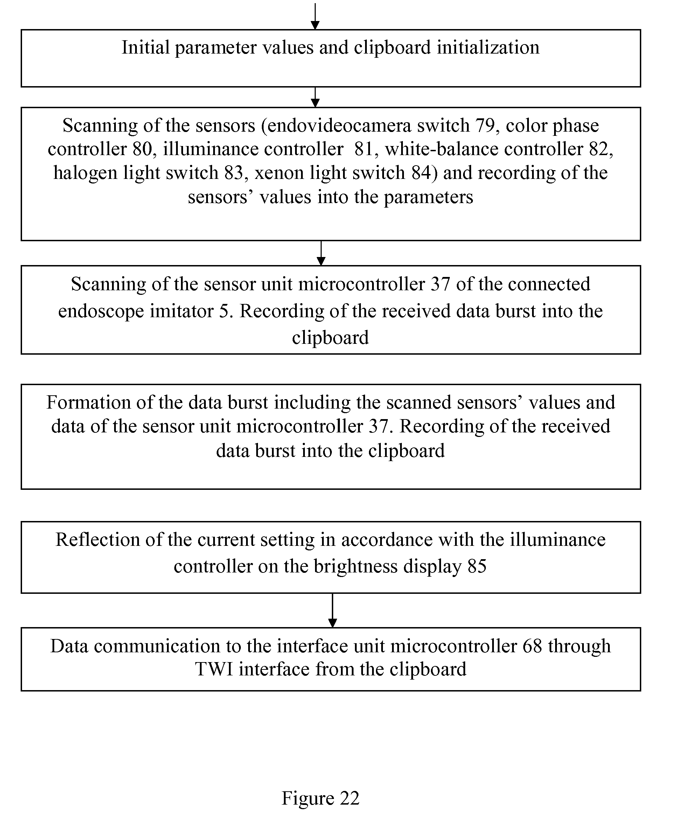

Let us consider the principal scheme of the endovideocamera control unit 6 coupled to the sensors unit 21 and interface unit 4 represented on FIG. 19, in operation. FIG. 22 represents a block diagram of the work algorithm of the microcontroller of the endovideocamera control unit 70. The microcontroller of the endovideocamera control unit 70 is located in the endovideocamera control unit 6. Data from the endovideocamera switch 79, color phase controller 80, illuminance controller 81, white-balance controller 82, halogen light switch 83, and xenon light switch 84 are being communicated to the microcontroller of the endovideocamera control unit 70. Data from the sensor unit microcontroller 37 located in the endoscope imitator 5 that is the laparoscopic instrument imitator are being communicated to the microcontroller of the endovideocamera control unit 70 through TWI interface. The microcontroller of the endovideocamera control unit 70 is communicating the collected data to the interface unit microcontroller 68 through TWI interface. On pushing the illuminance controller 81 the setting values are being displayed on the brightness display 85.

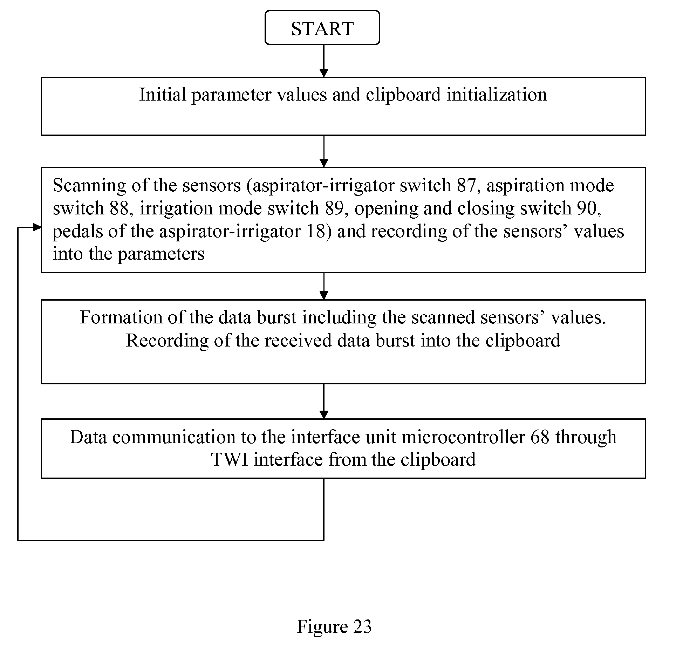

Let us consider the principal scheme of the aspirator-irrigator control unit 8 coupled to the interface unit 4 represented on FIG. 20, in operation. FIG. 23 represents a block diagram of the work algorithm of the microcontroller of the aspirator-irrigator control unit 71. The microcontroller of the aspirator-irrigator control unit 71 is located inside the aspirator-irrigator control unit 8. Data from the aspirator-irrigator switch 87, the aspiration mode switch 88, the irrigation mode switch 89, the opening and closing switch 90, and aspirator-irrigator pedals 18 are being communicated to the microcontroller of the aspirator-irrigator control unit 71. The microcontroller of the aspirator-irrigator control unit 71 is communicating the collected data to the interface unit microcontroller 68 through TWI interface.

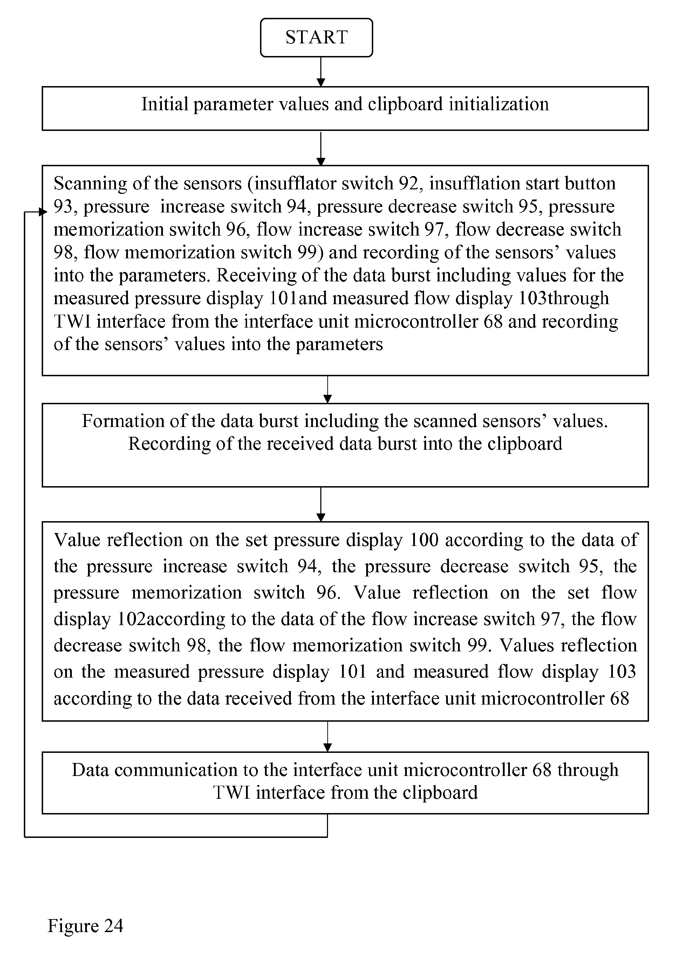

Let us consider the principal scheme of the insufflator control unit 10 coupled to the interface unit 4 represented on FIG. 21, in operation. FIG. 24 represents a block diagram of the work algorithm of the microcontroller of the insufflator control unit 72. The microcontroller of the insufflator control unit 72 is located inside the insufflator control unit 10. Data from the insufflator switch 92, insufflation start button 93, pressure increase switch 94, pressure decrease switch 95, pressure memorization switch 96, flow increase switch 97, flow decrease switch 98, and flow memorization switch 99 are being communicated to the insufflator control unit microcontroller 72. The insufflator control unit microcontroller 72 is communicating the received data to the interface unit microcontroller 68 through TWI interface.

Data received from the interface unit 68 are being displayed on the measured pressure display 101 and measured flow display 103. On pushing the pressure increase switch 94, the pressure decrease switch 95, and the pressure memorization switch 96, the setting values are being displayed on the set pressure display 100. On pushing the flow increase switch 97, flow decrease switch 98, and flow memorization switch 99, the setting values are being displayed on the set flow display 102.

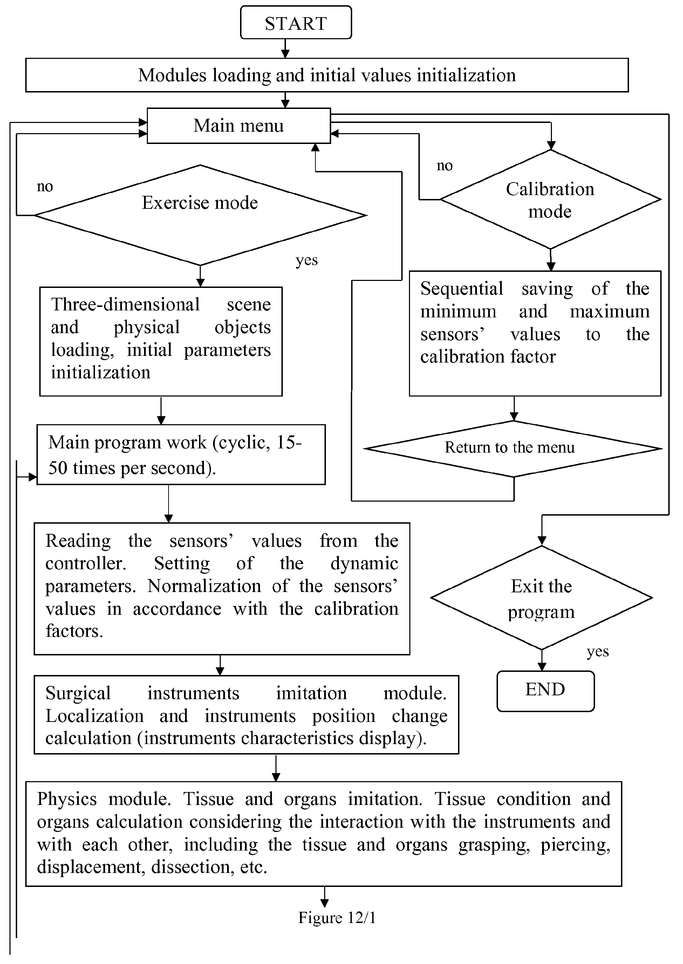

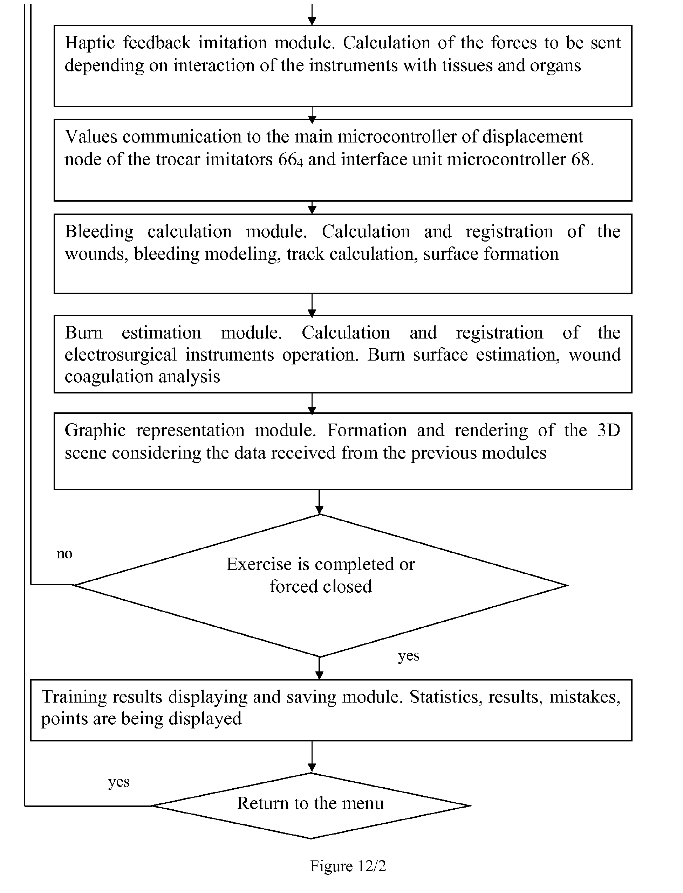

Let us consider the hybrid medical laparoscopic simulator represented on FIG. 1, in operation. On operating system loading execution of the program algorithm represented on block diagram FIG. 12, starts on the processor of the ECM 2 (FIG. 12 block diagram is represented on 2 pages, on the first page by the name of FIG. 12/1, on the second by the name of FIG. 12/2). In accordance with the algorithm represented on FIG. 12 block diagram, the graphic representation module displays a menu in the visualization system 12, where an exercise, a calibration mode or an exit is to be chosen. Choice of the menu options is performed with the manipulator of the ECM 2.

Manipulations with the laparoscopic instruments imitators performed by a surgeon are tracked by the encoder 24 and the Hall sensor 35 represented on FIG. 3 drawing by the instrument wing nut 23 rotation and handle 22 pressure. These sensors are coupled to the ECM 2 in accordance with the principal scheme of coupling the sensor unit microcontroller 37 of the laparoscopic instrument imitators with the sensors of imitator of the laparoscopic instrument represented on FIG. 8 and the principal scheme of the interface unit 4 coupled to the sensor unit 21 and control units represented on FIG. 9. Data from these sensors are being communicated to the ECM 2 in accordance with the work algorithm of the sensor unit microcontroller 37 of the laparoscopic instrument imitator represented on the block diagram FIG. 13 and the work algorithm of the interface unit microcontroller 68 represented on FIG. 14 block diagram.

Manipulations with the trocar imitators 11 with the help of the inserted laparoscopic instruments imitators are tracked with: instrument longitudinal motion detection sensor 42, Y-direction rotation sensor 46, and X-direction rotation sensor 49. Displacement of the trocar imitator 11 is tracked in the displacement node of the trocar imitator with the guideway longitudinal motion sensor 55 and the guideway rotation sensor 58. These sensors are coupled to the ECM 2 in accordance with the principal scheme of coupling the microcontrollers of displacement nodes of the trocar imitators 66 to the ECM 2 represented on FIG. 10. Data from these sensors are being communicated to the ECM 2 in accordance with the work algorithm of the microcontrollers of displacement nodes of the trocar imitators 66₁-66₃ represented on FIG. 15 block diagram and the work algorithm of the displacement node of the trocar imitator 66₄ represented on FIG. 16 block diagram.

The algorithm being executed on the processor of the ECM 2 connecting the manipulations of a surgical team with the image of the visualization system 12 is described below.

Let us consider the hybrid medical laparoscopic simulator considering the execution of the program on the processor of the ECM 2. In accordance with the algorithm represented on FIG. 12 block diagram, the graphic representation module in calibration mode generates into the visualization system 12 a signal with the dialog box image where the calibrated sensors are represented: encoder 24, instrument longitudinal motion detection sensor 42, Y-direction rotation sensor 46, X-direction rotation sensor 49, guideway longitudinal motion sensor 55, and guideway rotation sensor 58.

The laparoscopic instrument imitator is being inserted in the trocar imitator 11 and the minimum and maximum value of each calibrated sensor is being fixed sequentially. For instance, first of all for the instrument longitudinal motion detection sensor 42 calibration, the laparoscopic instrument imitator is inserted in the trocar imitator 11 all the way in, an appropriate minimum position fixation mode of the sensor is chosen in the dialog box with the manipulator of the ECM 2, then, the laparoscopic instrument imitator is taken-out incompletely from the trocar imitator 11 and an appropriate maximum position fixation mode of the sensor is chosen in the dialog box. Values of the calibration factors are saved by the program to the data base of the EMC 2 automatically in accordance with the algorithm represented on FIG. 12 block diagram and are used for normalization of signal values incoming from the corresponding sensors. Calibration could be remade until the complete match of the real movement of the instruments imitators inserted in the trocar imitators 11 with the virtual instruments reflected by the visualization system 12 in the exercises being performed.

Surgical team actions while working with the hybrid medical laparoscopic simulator are following: a nurse gives and takes instruments of a surgeon and his/her assistant at their request, connects the laparoscopic instrument imitators to the corresponding control units if necessary, aligns the equipment according to the surgeon's request, and provides support to the surgeon and his/her assistant. The surgeon guides the intervention process and accepts important decisions. The assistant holds the endoscope imitator 5 with one hand and the laparoscopic grasper 3 by the other hand as a rule, helping the surgeon to hold the virtual organs for comfortable execution of the intervention. Extra specialists' participation is possible on inserting in the simulator other types of operating room equipment, laparoscopic unit imitator, and other units. For instance, the participation of an anesthesiologist is necessary when an imitator of a stand type anesthetic unit is on.

3D models of the tissues and organs are simulated in *Autodesk 3ds Max* system, developed by *Autodesk* company, inserted in the data base of the ECM 2. According to the algorithm represented on FIG. 12 block diagram, the physics module is software developed with use of *SDK PhysX* of *nVidia* company. According to the algorithm represented on FIG. 12 block diagram, the graphic representation module is software developed with use of SDK DirectX of Microsoft company.

The description of the *Cholecystectomy* intervention training performance procedure on the hybrid medical laparoscopic simulator considering the program being performed on the processor of the ECM 2 in accordance with the algorithm in exercise mode represented on FIG. 12 block diagramis as follows: visualization system 12 reflects a chosen virtual patient's clinical case description in the form of the text information. The clinical case description includes case records and current complaints of the virtual patient. For instance, a 29-year-old female with primary cholecystitis, gallstone, pain in epigastrium shooting up the right shoulder girdle; a 43-year-old female with acute pain in the right hypochondrium after greasy food, ultrasonography shows an echogenic lesion in gall-bladder; a 37-year-old male presented with paroxysmal abdominal pain occurred in 5 minutes after lunch; a 48-year-old female presented with pain in the right hypochondrium, the pain is intermittent, occurring last few years, the analysis result shows increased level of bilirubin; a 52-year-ild male with overweight, complains of nausea and vomiting, pain in the right hypochondrium when palpated, liver is palpated; a 44-year-old female with overweight complains of nausea, pains presents during the last 12 hours and is growing when coughing and moving; a 46-year-old female with permanent pain in the right hypochondrium, dark-colored urine and feces, icteric sclerae; and a 49-year-old female with fever and chills, pain in the right hypochondrium, complains of nausea during the last 24 hours, sclerae and skin are icretic. According to the presented information, the surgeon has to decide how to place the robot-patient 1 on the operating table 14 and location of the trocar imitators 11, for example, considering the cicatrization following the previous interventions. The robot-patient 1 is being located on the operating table 14. The position of the first trocar imitator 11 where the endoscope imitator 5, which is the laparoscopic instrument imitator, is to be inserted is being chosen by the weakening of the displacement node wing nut 67, the position of the trocar imitator 11 in the abdominal cavity of the robot patient 1 relevant to the position during the real intervention is being set, then the displacement node wing nut 67 is being tightened.

The surgeon connects the insufflator pipe 9 to the trocar valve 53 on the insufflators control unit 10, and the nurse (or the surgeon) sets the pressure value with the pressure increase switch 94 and the pressure decrease switch 95 and presses the pressure memorization switch 96 over-watching the set pressure display 100. The endoscope imitator 5, which is a laparoscopic instrument imitator, is being inserted. The inserted endoscope imitator 5 through the infrared LED 36 sends the signal with the instrument code to the infrared detector 52 of the trocar imitator 11. Data on the instrument code and data of the position and orientation sensors (instrument longitudinal motion detection sensor 42, Y-direction rotation sensor 46, X-direction rotation sensor 49, guideway longitudinal motion sensor 55, and guideway rotation sensor 58) of the trocar imitator 11 are being communicated to the ECM 2. According to the instrument code, the program detects that the endoscope imitator 5 or any other laparoscopic instrument imitator was inserted. Data on the position and orientation of the trocar imitator 11 are being standardized considering the calibration factors which should be defined in the calibration mode in accordance with the algorithm represented on FIG. 12 block diagram. After the selection of the first trocar imitator 11 and installation of the endoscope imitator 5, which is a laparoscopic instrument imitator, the visualization system 12 displays the video data generated by the graphic representation module according to the position of the virtual endoscope by the algorithm represented on FIG. 12 block diagram. The nurse sets up the image quality on the endovideocamera control unit 6. The surgeon examines virtual organs and based on the received video data installs the other trocar imitators 11, gives the endoscope imitator 5 to the assistant, weakens the displacement node wing nut 67 for each trocar imitator 11, sets the necessary position of the trocar imitators 11, fixes the displacement node wing nut 67, and inserts the corresponding laparoscopic instrument imitator.

By the algorithm represented on FIG. 12 block diagram, the physics module simulates the physical properties of the laparoscopic instrument imitator on insertion of the each laparoscopic instrument imitator in the trocar imitator 11: firmness, volume, electrical discharges, extravasations, cutting surfaces, strains, space position, and interaction with the virtual organs and tissues; by the algorithm represented on FIG. 12 block diagram, the graphic representation module generates the image of each virtual laparoscopic instrument among the virtual organs and tissues according to the position of the virtual laparoscopic instruments.

Depending on the selected virtual patient and by the algorithm represented on FIG. 12 block diagram, the physics module simulates the physical properties of the virtual organs analogous to the real ones and forms the anatomic variant including the size, form, color, location of a gall-bladder, bile ducts, arteries, etc.

In this regard, one of the variants of the 3D computer model of the organs is being loaded from the database of the ECM 2 including the description of the organ surface (the surface is set by the points in 3D space, point surface normals, and connections between the points), of the textures (visual reflection of the imitated organs superimposed onto the 3D surface), and of the properties (elasticity, fragility, etc). After that, the conversion of the loaded data into the structures necessary for deformation and dissection simulation is performed.

*Cholecystectomy* intervention training could be performed both stage-by-stage and completely.

Stage-by-stage mode. Separate training of a selected intervention stage (traction, Calot's triangle preparation, clipping and the transection of the cystic duct and artery, and gall-bladder mobilization). By the algorithm represented on FIG. 12 block diagram, the physics module initializes in the database of the ECM 2 the physical models of organs in conditions relevant to the completed stages previous to the selected stage and sends a signal to the graphic representation module forming the 3D image of the virtual organs being sent to the visualization system 12. For this, the physics module modifies the 3D surfaces simulating the organs (for example, the performed traction, dissection and clips applied) by the algorithm represented on FIG. 12 block diagram.

When all the actions required for the selected stage are carried out, the automatic exit is performed.

Complete intervention procedure. By the algorithm represented on FIG. 12 block diagram, the physics module initializes in the database of the ECM 2 the physical models of organs in initial conditions (as they are located and deformed in the body of a real patient being laid on the operation table) and the graphic representation module forms the 3D image being sent to the visualization system 12 considering the data received from the physics module and other modules represented on FIG. 12 block diagram. By the algorithm represented on FIG. 12 block diagram, the logic module defines the attributes of the start and end of the next stage including the definition of the intervention stages failure. The exercise is over when all the intervention stages are completed.

### Intervention stages:

Traction. First of all, the surgeon or assistant should perform the traction - gallbladder virtual model displacement for provision of an access to the area being operated. The assistant inserts the laparoscopic grasper imitator 3 in the trocar imitator 11, performs manipulations with the laparoscopic grasper imitator 3 displayed in the visualization system 12 in the form of a virtual instrument, captures the gallbladder fundus, and displaces the virtual gallbladder with a laparoscopic grasper imitator 3, herein the physics module figures out the deformation of the 3D surface of the virtual gallbladder and its influence on the other virtual organs. The physics module estimates the current condition of the virtual organs surfaces a few times per second and the graphic representation module generates a signal incoming to the visualization system 12. As a result, there is a simulated image relevant to the orientation of the inserted endoscope imitator 5, which is a laparoscopic instrument imitator, with the image of a real mobility, smoothness of motion, and mutual influence of the virtual organs.

The logic module defines the accuracy of the traction direction (motion pattern is arranged on grounds of the calculated coordinates of the laparoscopic instrument imitator, the pattern is compared to the sample variant stored in the database of the ECM 2, and permissible variations are considered), the correctness of the area being operated visualization (the location of the virtual gallbladder and virtual vessels is estimated on the base of the coordinates of the surface points of the virtual organs and is compared to the sample variant stored in the database of the ECM 2, and permissible variations are considered), and correctness of the virtual organs grasp (the area of the grasp with the laparoscopic instruments is being estimated, the correspondence of the grasping zone to the permissible limits, and the instrument code is being compared to the allowed instrument codes stored in the database of the ECM 2).

Calot's triangle preparation. The surgeon has to dissect the abdominal membrane and the adipose tissue to allocate (to visualize) the tubular formations (artery and duct) to be clipped and transected. For this purpose, the surgeon inserts one of the laparoscopic instruments imitator, for instance, a dissector imitator and a hook imitator which are the laparoscopic instruments imitators, and the nurse hooks up the connector of the instrument to the coagulator control unit 13 and sets up the necessary mode and rate of the current with the corresponding switches (coagulation mode switch 75, cutting mode switch 76, a blend mode switch, and coagulator's current power controller 74) according to the surgeon's request. The values of the mode and rate of the currents are being communicated to the ECM 2. When manipulating the laparoscopic instrument imitator, the surgeon grasps the virtual adipose tissue, pulls it back, then dissects by pressing the pedal of coagulator 17 (the signal of the pressing the pedal of coagulator 17 is received by the ECM 2, and the physics module generates the virtual current flow through the virtual tissues). The physics module figures out what areas of the tissue are in contact with the virtual branches according to the coordinates of the virtual branches of the virtual instrument and coordinates of the surface points of the virtual tissue, and if the contact condition is fulfilled then the current flow through these areas of the tissue will be imitated, in the visualization system 12 the smoke and particles of the dissected tissue moving randomly are reflected, for the period of 1-3 seconds the full dissection of the contiguous areas of the virtual tissue, at that they disappear smoothly on the image of the visualization system 12. The logic module defines the correctness of the dissection direction (a track is laid out according to the average coordinates of the areas of the virtual tissue dissected sequentially and is compared for the similarity to the sample variants of the tracks, permissible variations are considered), meeting the safety requirements while handling the instrument (motion direction of the virtual branches of the virtual instrument from the grasp of the virtual tissue to the dissection start, the direction is being compared to the allowed one, permissible variations are considered), etc.

Clipping and transection of the artery and duct. The surgeon inserts the laparoscopic clip-applier imitator in the trocar imitator 11, manipulates it and achieves the entry of a necessary part of the virtual vessel between the branches of the virtual clip-applier by moving the virtual instrument, and applies the clip by clenching the handle of the laparoscopic clip-applier imitator. The physics module figures out the deformation of the vessel model, modifies the 3D vessel surface, and visualizes the applied clip in the place of its installation (the the position in the virtual environment and corresponding deformation of the virtual vessel is being figured out for each clip). After installation of the enough quantity of the clips, the surgeon inserts laparoscopic scissors imitator, manipulates it and approaches the incision site of the virtual vessel with the branches of the virtual scissors, and performs the incision. The physics module figures out changes of the 3D surface of the virtual vessel as a result of the virtual incision (the graphic representation module reflects the changes in the visualization system 12). The logic module defines the correctness of the applied clip location (the application area is being compared to the sample variants of the allowed application area), the quantity of the clips, and the correctness of the places of tubular formations transection (the transaction area is being compared to the sample variant of the allowed transaction areas) by the algorithm represented on FIG. 12 block diagram and description of the actions mentioned below.

Gallbladder mobilization. The surgeon has to separate the gallbladder from the liver. For this purpose, the surgeon inserts the hook imitator, and the nurse hooks up the connector of the instrument to the coagulator control unit 13 and sets up the necessary mode and rate of the current with the corresponding switches (coagulation mode switch 75, cutting mode switch 76, blend mode switch and coagulator's current power controller 74) according to the surgeon's request. The surgeon inserts the laparoscopic grasper imitator 3 in the second trocar imitator 11 (for the left hand), manipulates it, and grasps and pulls back the virtual gallbladder (the physics module figures out the corresponding surface deformations of the gallbladder and movement and deformation of other virtual organs depending on the movement of the virtual laparoscopic instruments). The surgeon manipulates the laparoscopic instrument imitators, brings the virtual hook to the adhesion place of the virtual gallbladder and the virtual liver, and presses the pedal of the coagulator 17. The physics module figures out if the tissue is in contact with the electrode of the virtual instrument according to the coordinates of the virtual instrument, and if the contact condition is fulfilled then the current flow through the contiguous area of the tissue will be imitated, in the visualization system 12 the smoke and particles of the dissected tissue moving randomly are reflected, for the period of 1-3 seconds the full dissection of the contiguous areas of the tissue, the 3D surfaces of the virtual liver and gallbladder are being detached that is visible in the visualization system 12 as well. The logic module defines the correctness of the contact of the virtual instruments with the virtual organs and tissues by the surgeon's laparoscopic instrument imitators manipulations - contact time (the logic module compares the contact time to the minimum and maximum, if the contact time is less than the minimum level for the set capacity, then the detachment of the virtual tissues will not be performed, if the contact time is more than the maximum level for the set capacity, then the extensive necrosis will be imitated), the contact place (the physics module figures out the current impact area in 3D coordinates, the logic module checks the permissibility and the relevance of the instrument use in the given area), etc., blood coagulation performance (the logic module compares the contact area to the database of the virtual vessels under the virtual organs surface, as a result of piercing, dissection with the virtual instruments or under the action of dissector bleeding is generated, that is visible in the visualization system 12; if there is a virtual bleeding, the fact of the coagulation the bleeding points for hemostasis with the instrument will be checked), irrigation (the surgeon has to insert the aspirator-irrigator imitator 7, manipulate it, direct the virtual fluid steam to the virtual organs surface areas requiring the washout of the blood, bile and dead tissue, meanwhile the track is being figured out and the steam is being reflected, the steam impact to the surface areas is figured out, the obtained surface area is being communicated to the bleeding calculation module, the washout part is being defined, that is visible in the visualization system 12), and the aspiration of the gallbladder bed, etc.

Emergency situations. The logic module generates the emergency situations, for example, cardiac standstill, etc. Meanwhile, the signals changing the values in the visualization system 12 and/or audiovisual and mechanical vital signs of the robot-patient 1 are being sent. The surgical team has to identify the emergency situation and react to it properly. The logic module analyzes further actions of the surgical team (for example, if there is a cardiac standstill, the surgeon and the assistant will have to stop the intervention immediately, if there is a virtual bleeding, the blood points should be coagulated or damaged virtual vessels should be clipped, after that the laparoscopic instrument imitators should be extracted for further reanimation, the correctness of the actions is defined in accordance with the inserted laparoscopic instrument imitators and presence of the non-arrested bleeding, timeliness is defined by means of a virtual timer).

The description of the *Adnexectomy* intervention (uterine appendages removal) training performance procedure on the hybrid medical laparoscopic simulator considering the program being performed on the processor of the ECM 2 in accordance with the algorithm in exercise mode represented on FIG. 12 block diagram to be as follows: visualization system 12 reflects a chosen virtual patient's clinical case description of a in the form of the text information. The clinical case description includes case records, current complaints of the virtual patient. For instance, a 43-year-old female, whose ultrasonography shows a tumorous cavitary lesion, is presented with acute pain in iliac region (clinical case of oothecoma torsion); and a 30-year-old female is admitted to the hospital with acute lower abdominal pain in the left inguinal region, diagnostic laparoscopy due to the left appendages torsion is in the past history, detorsion of the left appendages with their preserving is performed.

According to the presented information, the surgeon has to decide how to place the robot-patient 1 on the operating table 14 and location of the trocar imitators 11, for example, considering the cicatrization following the previous interventions. The robot-patient 1 is being located on the operating table 14. The position of the first trocar imitator 11 where the endoscope imitator 5, which is the laparoscopic instrument imitator, is to be inserted is being chosen by the weakening of the displacement node wing nut 67, the position of the trocar imitator 11 in the abdominal cavity of the robot patient 1 relevant to the position during the real intervention is being set, then the displacement node wing nut 67 is being tightened. The surgeon connects the insufflator pipe 9 to the trocar valve 53 on the insufflators control unit 10, and the nurse (or the surgeon) sets the pressure value with the pressure increase switch 94 and the pressure decrease switch 95, and presses the pressure memorization switch 96 over-watching the set pressure display 100. The endoscope imitator 5, which is a laparoscopic instrument imitator, is being inserted. The inserted endoscope imitator 5 through the infrared LED 36 sends the signal with the instrument code to the infrared detector 52 of the trocar imitator 11. Data on the instrument code and data of the position and orientation sensors (instrument longitudinal motion detection sensor 42, Y-direction rotation sensor 46, X-direction rotation sensor 49, guideway longitudinal motion sensor 55, and guideway rotation sensor 58) of the trocar imitator 11 are being communicated to the ECM 2. According to the instrument code, the program detects that the endoscope imitator 5 or any other laparoscopic instrument imitator was inserted. Data on the position and orientation of the trocar imitator 11 are being standardized considering the calibration factors which should be defined in the calibration mode in accordance with the algorithm represented on FIG. 12 block diagram.

After the selection of the first trocar imitator 11 and installation of the endoscope imitator 5, which is a laparoscopic instrument imitator, the visualization system 12 displays the video data generated by the graphic representation module according to the position of the virtual endoscope by the algorithm represented on FIG. 12 block diagram. The nurse sets up the image quality on the endovideocamera control unit 6. The surgeon examines virtual organs and on the base of the received video data installs the other trocar imitators 11, gives the endoscope imitator 5 to the assistant, weakens the displacement node wing nut 67 for each trocar imitator 11, sets the necessary position of the trocar imitator 11, fixes the displacement node wing nut 67 and inserts the corresponding laparoscopic instrument imitator. By the algorithm represented on FIG. 12 block diagram, the physics module simulates the physical properties of the laparoscopic instrument imitator on insertion of the each laparoscopic instrument imitator in the trocar imitator 11: firmness, volume, electrical discharges, extravasations, cutting surfaces, strains, space position, and interaction with the virtual organs and tissues. By the algorithm represented on FIG. 12 block diagram, the graphic representation module generates the image of each virtual laparoscopic instrument among the virtual organs and tissues according to the position of the virtual laparoscopic instruments.

Depending on the selected virtual patient and by the algorithm represented on FIG. 12 block diagram, the physics module simulates the physical properties of the virtual organs analogous to the real ones and forms the anatomic variant, including the size, form, color, location of uterus, fallopian tubes, ovaries, ligaments, mesosalpinx, etc. In this regard one of the variants of the 3D computer model of the organs is being loaded from the database of the ECM 2 including the description of the organ surface (the surface is set by the points in 3D space, point surface normals, and connections between the points), and of the textures (visual reflection of the imitated organs superimposed onto the 3D surface), of the properties (elasticity, fragility, etc). After that the conversion of the loaded data into the structures necessary for deformation and dissection of the organs (tissues) simulation is performed.

*Adnexectomy* intervention training could be performed both stage-by-stage and completely.

Stage-by-stage mode. Separate training of a selected intervention stage (traction, mesosalpinx coagulation and its dissection, and hemostasis control). By the algorithm represented on FIG. 12 block diagram, the physics module initializes in the database of the ECM 2 the physical models of organs in conditions relevant to the completed stages previous to the selected stage and sends a signal to the graphic representation module forming the 3D image of the virtual organs being sent to the visualization system 12. For this the physics module modifies the 3D surfaces simulating the organs (for example, the performed traction, dissection and clips applied) by the algorithm represented on FIG. 12 block diagram. When all the actions required for the selected stage are carried out, the automatic exit is performed.

Complete intervention procedure. By the algorithm represented on FIG. 12 block diagram, the physics module initializes in the database of the ECM 2 the physical models of organs in initial conditions (as they are located and deformed in the body of a real patient being laid on the operation table) and the graphic representation module forms the 3D image being sent to the visualization system 12 considering the data received from the physics module and other modules represented on FIG. 12 block diagram. By the algorithm represented on FIG. 12 block diagram, the logic module defines the attributes of the start and end of the next stage including the definition of the intervention stages failure. The exercise is over when all the intervention stages are completed.

### Intervention stages:

Traction. First of all, the surgeon or assistant should perform the traction - virtual organs model displacement for provision of an access to the area being operated. The assistant inserts the laparoscopic grasper imitator 3 in the trocar imitator 11, performs manipulations with the laparoscopic grasper imitator 3 displayed in the visualization system 12 in the form of a virtual instrument, captures a distal end of the fallopian tube with a laparoscopic grasper imitator 3, lifts it headward and sideways a little. They examine the mesosalpinx location, ovary ligaments, approximately identify the ureter duct (in this regard virtual organs and tissues are being displaced carefully with the laparoscopic instrument imitator, receive the image where the structure of the organs and tissues is clear, herein the physics module figures out the deformation of the 3D surface that is reflected by the graphic representation module in the visualization system 12).

The logic module defines the accuracy of the traction direction (motion pattern is arranged on grounds of the calculated coordinates of the laparoscopic instrument imitator, the pattern is compared to the sample variant stored in the database of the ECM 2, and permissible variations are considered), rough or excessive displacement of the virtual fallopian tube (the logic module figures out the displacement speed and distance on the basis of the change of the laparoscopic instrument imitator coordinates, being communicated few times per second (20-50), meanwhile the physics module figures out the 3D surface deformation that is reflected by the graphic representation module in the visualization system 12, calculates the stretch ratio, the logic module compares it the sample variant stored in the database of the ECM 2), incorrect overlapping of the laparoscopic grasper imitator 3 (the physics module figures out the overlapping area on the basis of the virtual laparoscopic grasper's branches coordinates, the logic module compares it the sample variant stored in the database of the ECM 2), incorrect instrument choice (the logic module compares the instrument code to the allowed instrument codes in the database of the ECM 2), damage of the abdominal membrane, ureter, branch of an arterial or venous vessel, hollow organ, urinary bladder, intestines by adhesiotomy (the physics module figures out the distance to the different areas of the 3D surface of the virtual organs and tissues for damage definition, the logic module compares it to the minimum level during the work of the virtual instrument and figures out the impact pressure of the virtual instrument to the 3D surface of the virtual organs according to the received coordinates of the laparoscopic instruments imitator, compares it to the maximum permissible pressure limit for the surface area; virtual damages are being saved in the database of the ECM 2 and are being communicated to the bleeding calculation module and logic module, that is being reflected by the graphic representation module in the visualization system 12).

Mesosalpinx coagulation and dissection. The surgeon has to coagulate and dissect the virtual mesosalpinx, pulled back with the virtual laparoscopic grasper, overlapped to the tube, its edge, the lateral and bottom edge of the virtual ovary. The surgeon inserts the laparoscopic grasper imitator 3 to the one trocar imitator 11, and the coagulator imitator to the other one. The surgeon grasps and pulls back the virtual mesosalpinx with the laparoscopic grasper imitator 3, overlaps the forceps of the virtual coagulator, presses the pedal of the coagulator 17, and holds down for 1-2 seconds. The physics module figures out the coagulation area according to the virtual forceps coordinates and the logic module figures out the power and contact time. The graphic representation module reflects the yellowing or discoloration of the tissue around the forceps of the virtual coagulator and saves the changed characteristics of the virtual tissues to the database of the ECM 2. After coagulation of each small area, the surgeon takes out the coagulator imitator and inserts the laparoscopic scissors imitator instead of it, approaches the branches 19 of the laparoscopic scissors imitator, the actions of which are coordinated with the virtual ones, to the coagulated area of the virtual tissue, and dissects it pressing the handle 22 that is reflected in the visualization system 12 (the physics module figures out the dissection area on the base of the coordinated of the virtual branches at the moment of dissection, the relevant alterations are made in the 3D surface, and data are saved into the database of the ECM 2.). Then it is necessary to coagulate and transect the utero-ovarian ligament and the infundibulopelvic ligament (is performed in the same way as mesosalpinx coagulation and transaction described above). The logic module figures out the incorrect lesion identification of the virtual fallopian tube, the utero-ovarian ligament, and the infundibulopelvic ligament (the logic module figures out the contact area and compares it to the sample variants stored in the database of the ECM 2when signals of coagulation or dissection of the virtual tissue areas arrive, if the contact areas do not coincide with the sample variants regarding the permissible deviations, the it will generate a text message coming through the graphic representation module to the visualization system 12 informing that the surgeon started the surgical intervention having identified the anatomical structures incorrectly), coagulation being performed too close to the pelvic walls (if the logic module identifies the crossing of the coagulation area with the surface area stored in the database of the ECM 2 as the virtual pelvic wall, or if the distance to this area is less than the minimum permissible level, then the error data will be saved to the database), and damage of the ureter (if the logic module identifies the crossing of the dissection area with the surface area where the virtual ureter is located, then the data of the ureter damage will be saved to the database of the ECM 2, that will be reflected by the graphical representation module in the visualization system 12).

Hemostasis control. The surgeon examines all the area being operated with the virtual endoscope coordinated to the real endoscope imitator 5. If any damages are found, then the surgeon will performed the coagulation in a manner described above. The logic module analyzes the examination for bleeding (the track of the virtual endoscope movement and stop time for every area of the area being operated are saved to the database of the ECM 2, the logic module compares it to the sample variants in the database considering the permissible deviations) and the damage of the organs nearby. If bleeding and damages are presented, the logic module checks the relevant action of the surgeon, for example bleeding coagulation (present virtual damages and bleedings are registered in the database of the ECM in accordance with the process of damage detection and registration described above, during the coagulation close to the damage coordinates at the distance less than the sample variant over 1-2 seconds *Arrest of bleeding* will be saved to the database, if some bleedings are present, then *Error* will be saved).

Therefore on the base of foregoing, the hybrid medical laparoscopic simulator provides the real position of the surgical team regarding a robot-patient 1 and operational site; opportunities of different options of laparoscopic access execution in case of complicated clinical situations according to the chosen position of the robot-patient 1; training of the team work of operating and assisting surgeons, and scrub nurse by the different scenarios; and usage of more than three trocar imitators 11, change of the quantity of the trocar imitators 11, choice of the location of the trocar imitators 11, reality of the different instrument imitators and their insertion in the trocar imitators 11.

### Industrial Applicability

The specialist could put the present solution into practice as it presented in the patent claim with stated results using the above description and drawings.

## Claims

1. A hybrid medical laparoscopic simulator comprising:
an ECM;
laparoscopic instrument imitators coupled to the ECM;
trocar imitators coupled to the ECM;
a visualization system coupled to the ECM attained by the presence of a robot-patient;
laparoscopic unit imitators coupled to the ECM; and
operating room equipment, wherein each of the laparoscopic instrument imitators is executed in a form of a real instrument, contains a sensor unit, and is separated from at least four trocar imitators installed in an abdominal cavity of the robot-patient,
each of the trocar imitators is coupled to a corresponding displacement node of the trocar imitator, and
a position of the trocar imitator on an anterior wall of the abdominal cavity of the robot-patient is traced and defined.
